# EUROPÄISCHE PATENTSCHRIFT

(11) **EP 1 802 302 B1**
(45) Veröffentlichungstag und Bekanntmachung des Hinweises auf die Patenterteilung: **26.10.2016**
(21) Anmeldenummer: 05784163.7
(22) Anmeldetag: 16.09.2005
(51) Int. Cl.: A61K 31/4174, A61P 31/16

(54) **NEUE VERWENDUNG FÜR alpha-SYMPATHOMIMETIKA MIT 2-IMIDAZOLINSTRUKTUR**
NOVEL USE OF alpha-SYMPATHOMIMETICS HAVING A 2-IMIDAZOLINE STRUCTURE
NOUVELLE UTILISATION D'(alpha)-SYMPATHOMIMETIQUES PRESENTANT UNE STRUCTURE 2-IMIDAZOLINE

(30) Priorität: 05.10.2004 DE 102004049008
(43) Veröffentlichungstag der Anmeldung: 04.07.2007
(73) Patentinhaber: Merck Patent GmbH, 64293 Darmstadt (DE)
(72) Erfinder: SACHER, Fritz, 55435 Gau-Algesheim (DE); TSCHAIKIN, Marion, 64297 Darmstadt (DE); KOELSCH, Stephan, 67549 Worms (DE)
(86) Internationale Anmeldenummer: PCT/EP2005/010026
(87) Internationale Veröffentlichungsnummer: WO 2006/037452

(56) Entgegenhaltungen:
- WO-A-96/25163
- WO-A-99/15203
- WO-A-03/024433
- US-B1- 6 423 724
- DATABASE WPI Section Ch, Week 199435 Derwent Publications Ltd., London, GB; Class A96, AN 1994-283256 XP002354491 & JP 06 211659 A (KOWA CO LTD) 2. August 1994 (1994-08-02)
- SACHER F ET AL: "The mechanism of the antiviral action of oxymetazoline" DEUTSCHE APOTHEKER ZEITUNG 03 FEB 2005 GERMANY, Bd. 145, Nr. 5, 3. Februar 2005 (2005-02-03), Seiten 111-112, XP001207744 ISSN: 0011-9857
- INGRID U SCOTT: 'Viral Conjunctivitis' 28 Januar 2013, Seiten 1 - 1, XP055052046

## Beschreibung

Die Erfindung betrifft α-Sympathomimetika mit 2-Imidazolinstruktur Oxymetazolin und Xylometazolin zur Verwendung zur Prophylaxe und/oder Behandlung viraler Erkrankungen, wobei Oxymetazolin und/oder Xylometazolin antiviral wirksam sind.

Oxymetazolin und Xylometazolin werden zur lokalen Behandlung von Schwellungen der Nasenschleimhaut, zum Beispiel bei Rhinitis acuta, Rhinitis allergica, eingesetzt. Oxymetazolin und Xylometazolin stimulieren α-Rezeptoren an den Gefäßen und wirken so stark vasokonstriktorisch. Bei lokaler Applikation in die Nase ergibt sich eine deutliche Konstriktion der Gefäße in der Nasenschleimhaut, die dann zu einer Verringerung der Schleimsekretion und zur Abschwellung der Schleimhäute führt. Dies verbessert die Luftdurchgängigkeit und beseitigt die Behinderung der Nasenatmung.

Rhinitis acuta, auch Schnupfen genannt, ist eine akute Nasenschleimhautentzündung, die durch Viren hervorgerufen wird. Die oben beschriebene Verwendung von Oxymetazolin und Xylometazolin bei Rhinitis acuta zur Schleimhautabschwellung beruht auf deren oben beschriebener vasokonstringierenden Wirkung. Ihre Verwendung bei der Behandlung der Rhinitis acuta ist somit nicht gegen die Erkrankung Rhinitis acuta an sich gerichtet, sondern ist lediglich eine palliative Behandlung, die gegen das auch bei Rhinitis acuta auftretende (unspezifische) Symptom Schleimhautschwellung gerichtet ist.

WO 99/15203 A1 offenbart pharmazeutische Zusammensetzungen enthaltend topische abschwellende Mittel zur Handhabung (Management) der allergischen und/oder vasomotorischen Rhinitis, Konjunktivitis, Erkältung, erkältungsähnlichen und/oder Grippesymptomen.

WO 03/024433 A2 offenbart die Verwendung von topischen sympathomimetischen abschwellenden Mittel (topical svmpathomimetic decongestant) wie eines Imidazolin-α-adrenergen Agonisten (der ausdrücklich als Vasokonstriktor ausgewiesen ist) wie Xylometazolin zur Erleichterung von nasaler Verstopfung (relief nasal congestion) und zur Vorbeugung von Sinusitis. Überraschenderweise wurde gefunden, dass Oxymetazolin und Xylometazolin antiviral wirksam sind und damit zur kausalen Prophylaxe und/oder Behandlung viraler Erkrankungen eingesetzt werden können. Gegenstand der Erfindung ist daher Oxymetazolin und/oder Xylometazolin zur Verwendung zur antiviralen Prophylaxe und/oder Behandlung viraler Erkrankungen ausgewählt aus der Gruppe bestehend aus Rhinitis acuta, Influenza, Parainfluenza, Otitis media und Sinusitis.

Das erfindungsgemäße, zur Prophylaxe und/oder Behandlung viraler Erkrankungen bestimmte Arzneimittel enthält Oxymetazolin und/oder Xylometazolin.

Besonders bevorzugt verwendet wird Oxymetazolin.

Virale Erkrankungen, zu deren Prophylaxe und/oder Behandlung Oxymetazolin und/oder Xylometazolin eingesetzt werden, sind Rhinitis acuta, Influenza, Parainfluenza, Otitis media und Sinusitis.

Das Oxymetazolin und/oder Xylometazolin enthaltende Arzneimittel kann vorbeugend, d. h. prophylaktisch verabreicht werden. In diesem Falle werden die die Erkrankung erregenden Viren sofort nach Befall bekämpft, sodass die Erkrankung erst gar nicht auftritt. Ebenso kann das Oxymetazolin und/oder Xylometazolin enthaltende Arzneimittel auch nach Ausbruch der Erkrankung eingesetzt werden, d. h. zur Bekämpfung der Viren, wenn diese bereits zur Erkrankung geführt haben, und damit zur Behandlung der viralen Erkrankung. Je nach Verwendung kann das Oxymetazolin und/oder Xylometazolin enthaltende Arzneimittel systemisch oder topisch verabreicht werden. Unter systemischer Applikation werden alle Applikationswege verstanden, die zur Folge haben, dass der/die Wirkstoff/e nach dessen Verabreichung vom Körper resorbiert und über die Blutbahnen im gesamten Körper verteilt werden. Dies sind insbesondere die orale und die parenterale Verabreichung, aber auch andere Applikationsarten wie z. B. die transdermale oder die pulmonale Applikation.

Unter topischer Applikation werden alle Applikationswege verstanden, mittels derer die den/die Wirkstoff/e enthaltenden Arzneimittel direkt auf Körperoberflächen oder in Hohlorgane, an oder in denen sie ihre Wirkung entfalten sollen, verabreicht werden. In Frage kommt insbesondere die Verabreichung auf die Haut einschließlich der Körperöffnungen auskleidenden Haut, wie z. B. im Ohr, oder auf Schleimhäute, beispielsweise am Auge, in der Nase, im Hals-Rachen- oder im Rektal-Bereich.

Die topische Applikation ist bevorzugt. Gegenstand der Erfindung ist daher Oxymetazolin und/oder Xylometazolin zur Verwendung zur antiviralen Prophylaxe und/oder Behandlung viraler Erkrankungen ausgewählt aus der Gruppe bestehend aus Rhinitis acuta, Influenza, Parainfluenza, Otitis media und Sinusitis, das dadurch gekennzeichnet ist, dass das Arzneimittel zur topischen Anwendung bestimmt ist.

Besonders bevorzugt ist die topische Anwendung des erfindungsgemäßen Arzneimittels auf Schleimhäuten. Gegenstand der Erfindung ist daher auch Oxymetazolin und/oder Xylometazolin zur Verwendung zur Prophylaxe und/oder Therapie viraler Erkrankungen ausgewählt aus der Gruppe bestehend aus Rhinitis acuta, Influenza, Parainfluenza, Otitis media und Sinusitis, das dadurch gekennzeichnet ist, dass das Arzneimittel zur Anwendung auf Schleimhäute bestimmt ist.

Der Einsatz des Arzneimittels zur antiviralen Prophylaxe und/oder Behandlung der Rhinitis acuta und/oder Influenza ist ganz besonders bevorzugt.

Es ist selbstverständlich, dass die Form des Arzneimittels dem Applikationsweg jeweils angepasst werden muss. Geeignete orale Darreichungsformen können z. B. Tabletten, Kapseln, Dragees, Granulate oder Liquida, Parenteralia z. B. Lösungen, Emulsionen oder Suspensionen, topische Darreichungsformen z. B. Gele, Salben, Lotionen, Cremes, Lösungen, Emulsionen, Suspensionen, Puder, Suppositorien oder Aerosole sein. Die für den jeweiligen Verwendungszweck geeigneten Applikationsformen, deren Zusammensetzung und Herstellung sind dem Fachmann wohlbekannt und bedürfen hier keiner weiteren Erläuterung. Verwiesen wird auf die einschlägigen Standardwerke, beispielsweise H. Sucker, P. Fuchs, P. Speiser, "Pharmazeutische Technologie", Stuttgart 1978 oder K.H. Bauer, K.H. Frömming, C. Führer, "Pharmazeutische Technologie", Stuttgart 1986. Diese werden hiermit als Referenz eingeführt und sind somit Teil der Offenbarung.

Die Oxymetazolin und/oder Xylometazolin können als Base oder auch als dessen Säureadditionssalze mit anorganischen Säuren z. B. Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure oder Sulfaminsäure, oder mit organische Säuren, wie z.B. Ameisensäure, Essigsäure, Pivalinsäure, Malonsäure, Bernsteinsäure, Pimelinsäure, Fumarsäure, Maleinsäure, Milchsäure, Weinsäure, Äpfelsäure oder Citronensäure eingesetzt werden. Besonders bevorzugt eingesetzt werden die Säureadditionssalze und hier wiederum die Hydrochloride. Dies gilt insbesondere dann, wenn wässrige Lösungen eingesetzt werden, die bevorzugt sind.

Nach einer vorteilhaften Ausführungsform der Erfindung wird ein Arzneimittel eingesetzt, das zusätzlich ein oder mehrere Zinksalze enthält. Als Zinksalze können grundsätzlich alle pharmazeutisch akzeptablen Zinksalze enthalten sein, bevorzugt sind Zinkchlorid, Zinklactat, Zinksulfat, Zinkcitrat, Zinkacetat, Zinkhistidinat, Zinkorotat, Zinkaspartat und/oder Zinkgluconat. Besonders bevorzugt enthält das Arzneimittel Zinkgluconat.

Nach einer bevorzugten Ausführungsform der Erfindung wird ein Arzneimittel eingesetzt, das dadurch gekennzeichnet ist, dass Oxymetazolin und/oder Xylometazolin in einem wässrigen Lösungsmittel enthalten ist/sind. Wässrige Lösungen sind beispielsweise besonders geeignet zur topischen Anwendung auf Schleimhäuten, wie z. B. am Auge im Hals-Nasen-Rachen- oder Rektal-Bereich und werden insbesondere bevorzugt in der Nase zur Behandlung der Rhinitis acuta eingesetzt.

Die wässrigen Lösungen können Hilfsstoffe wie z. B. Puffer und Konservierungsmittel enthalten. Als Puffer geeignet sind grundsätzlich alle physiologisch verträglichen Substanzen, die zur Einstellung des gewünschten pH-Wertes geeignet sind wie z. B. Citrat-Salze, Acetat-Salze, Histidin-Salze Succinat-Salze, Malat-Salze, Phosphat-Salze oder Lactat-Salze und/oder deren jeweilige freien Säuren bzw. Basen sowie Mischungen aus den verschiedenen Salzen und/oder deren Säuren bzw. Basen. Besonders bevorzugt enthalten wässrige Lösungen mit Oxymetazolin und/oder Xylometazolin Phosphat- oder Citratpuffer. Nach einer besonders bevorzugten Ausführungsform der Erfindung liegt das Arzneimittel, das Oxymetazolin und/oder Xylometazolin enthält und zur antiviralen Prophylaxe und/oder Behandlung viraler Erkrankungen vorgesehen ist als wässrige Lösung vor und enthält mindestens einen Puffer, vorzugsweise Phosphat- oder Citratpuffer.

Geeignete Phosphatpuffer sind Lösungen der Mono- und/oder Di-Natrium- und Kaliumsalze der Phosphorsäure, wie Dinatriumhydrogenphosphat oder Kaliumdihydrogenphosphat, sowie Mischungen der Natrium- und Kaliumsalze, wie beispielsweise Mischungen aus Dinatriumhydrogenphosphat und Kaliumdihydrogenphosphat.

Geeignete Citratpuffer sind Mischungen aus einem oder mehreren Citrat-Salz/en und/oder dessen freie Säure (z.B. Citronensäure, Citronensäure-Monohydrat, triNatriumcitrat-Dihydrat, tri-Kaliumcitrat-Monohydrat).

Der pH-Wert der wässrigen Lösung liegt im Bereich von 4,0 bis 8,0, bevorzugt ist ein pH-Wert von 5,5 bis 7,0. Soweit die wässrige Lösung Phosphatpuffer enthält, ist dieser zweckmäßigerweise in einer Konzentration von 5 bis 180 mmol/l enthalten, bevorzugt sind 140 bis 145 mmol/l. Soweit Citratpuffer enthalten ist, liegt dieser zweckmäßigerweise in einer Konzentration von 1 bis 50 mmol/l vor, bevorzugt sind 5 bis 20 mmol/l.

Geeignete Konservierungsmittel sind z. B. Phenol, m-Cresol, Methyl- oder Propylparaben, Chlorbutanol, Thiomersal oder Benzalkoniumchlorid, wovon Benzalkoniumchlorid, insbesondere wenn eine wässrige Lösung zur topischen Anwendung auf Schleimhäuten und hier insbesondere zu Anwendung in der Nase vorgesehen ist, bevorzugt ist. Nach einer weiteren bevorzugten Ausführungsform der Erfindung liegt das Arzneimittel, das Oxymetazolin und/oder Xylometazolin enthält und zur antiviralen Prophylaxe und/oder Behandlung viraler Erkrankungen vorgesehen ist als wässrige Lösung vor und enthält mindestens ein Konservierungsmittel, vorzugsweise Benzalkoniumchlorid.

Soweit die Lösung durch die osmotischen Eigenschaften des Wirkstoffs und der weiterhin enthaltenen Hilfsstoffe nicht bereits isotonisch ist, kann vorteilhaft weiterhin ein Isotonisierungsmittel, bevorzugt ein physiologisch verträgliches Salz, wie beispielsweise Natrium- oder Kaliumchlorid, oder ein physiologisch verträgliches Polyol, wie beispielsweise Glucose oder Glycerin, in einer zur Isotonisierung erforderlichen Menge enthalten sein. Als Isotonisierungsmittel besonders bevorzugt ist Glycerin.

Je nach enthaltenem α-Sympathomimetikum, nach Darreichungsform sowie in Abhängigkeit von der jeweiligen viralen Erkrankung kann das Arzneimittel zur Verabreichung von ein bis mehrmals wöchentlich bis ein bis mehrmals täglich bestimmt sein. Nach einer Ausführungsform der Erfindung ist das Arzneimittel zur zweimal, dreimal oder mehr als dreimal täglichen Verabreichung vorgesehen.

Die Beispiele, ohne hierauf beschränkt zu sein, erläutern die Erfindung.

### Beispiel

### Antivirale Aktivität

Die antivirale Aktivität der α-Sympathomimetika mit 2-Imidazolinstruktur wird in einer in-vitro Versuchsreihe am Beispiel Oxymetazolin gegenüber den Virus-Stämmen HRV 2 und HRV 14 (human rhino virus) sowie Influenza A untersucht. Die Untersuchungen erfolgen an mit HRV 2 bzw. HRV 14 infizierten HeLa-Zellen bzw. mit Influenza A infizierten MDCK-Zellen. Hier wird der Einfluss von Oxymetazolin auf die Virusreplikation/Virusvermehrung durch das Plaquerduktionssassay (siehe Cooper, P.D., 1955: A method for producing plaques in agar suspensions of animal cells. Virologiy 1:397-409) sowie auf die Infektiosität der Viren (Bestimmung der Restinfektiosität durch Virustitration) nachgewiesen. Daneben wurden antivirale Wirkeigenschaften durch das sogen. "high-throughput cytopathic effect inhibitory assay" (CPE/zpE) gemessen (Schmidtke M., Schnittler U., Jahn B., Dahse H.-M. and Stelzner A. 2001: A rapid assay for evaluation of antiviral activity against coxsackie virus B3, influenza virus A, and herpes simplex virus type 1. J Virol Methods 95: 133-143).

### Virus-Stämme HRV 2 und HRV 14

Zum Ausschluss von durch den Wirkstoff bedingten zelltoxischen Wirkungen wird zunächst durch Zugabe einer Verdünnungsreihe wässriger Wirkstoff-Lösungen die Wirkstoffkonzentration ermittelt, bei der sich unter den gegebenen in vitro Testbedingungen kein Einfluss auf die enthaltenen HeLa-Zellen ergibt (siehe Mosmann, T., 1983: Rapid Colorimetric Assay for Cellular Growth and Survival: Application to Proliferation and Cytotoxicity Assays. J. Immunol.Meth. 65:55-63). Die Wirkstoffkonzentration, bei der sich keine durch die Testsubstanzen bedingten, den Stoffwechsel der HeLa-Zellen reduzierenden Wirkungen ergeben, beträgt zwischen 0,005 und 0,003125%(G/V).

Die Infektion der HeLa-Zellen mit HRV 14 erfolgt mit einer mittleren Infektionsdosis (M.O.I., multiplicity of infection) von 0,0004. Für die Infektion der HeLa-Zellen mit HRV 2 wurde eine Virusdosis gewählt, die 72 Stunden nach der Infektion zu einem vollständigen zpE in den unbehandelten Viruskontrollen führte. Die Testsubstanz wird in Verdünnungen (1:2, 1:4, 1:8, 1:16, 1:32) von der ermittelten, nicht zelltoxischen Substanzkonzentration von 0,003125 % (G/V) zu den infizierten Zellen zugegeben. Die antivirale Aktivität wird mit Hilfe des Plaquereduktionstest und/oder dem "high-throughput cytopathic effect inhibitory assay" (CPE/zpE) gemessen. Die Restinfektiosität (TCID₅₀/ml) wird mittels Virustitration ermittelt. Die Ergebnisse HRV 14 sind in den Tabellen 1 und 2 zusammengestellt.

### HRV 14

**Tabelle 1**

| Verdünnung (1/X) | Plaquereduktion (A), rel. Hemmung [%] | Restinfektiosität (B), rel. Hemmung [%] | rel. Gesamthemmung (A) / (B) |
|---|---|---|---|
| 2 | 44,26 | 33,33 | 38,79 |
| 4 | 32,98 | 20,83 | 26,91 |
| 8 | 25,74 | 18,75 | 22,25 |
| 16 | 17,66 | 18,75 | 18,20 |
| 32 | 14,26 | 12,50 | 13,38 |

Die Ergebnisse zeigen beispielhaft für Oxymetazolin die antivirale Wirkung der α-Sympathomimetika mit 2-Imidazolinstruktur (hier gegenüber dem Virusstamm HRV 14).

**Tabelle 2**

| Verdünnung (1/X) | ZpE-Hemmung [%] |
|---|---|
| 2 | 50,5 |
| 4 | 53,6 |
| 8 | 37,5 |
| 16 | 5,9 |

Ergebnisse einer zweiten Versuchsreihe mit dem Virusstamm HRV 14 (Messung mittels CPE-Assay).

Die Ergebnisse für HRV 2 sind in der Tabelle 3 zusammengestellt.

### HRV 2

**Tabelle 3**

| Verdünnung (1/X) | ZpE-Hemmung [%] |
|---|---|
| 2 | 40,5 |
| 4 | 23,2 |

Die Ergebnisse zeigen beispielhaft für Oxymetazolin die antivirale Wirkung der α-Sympathomimetika mit 2-Imidazolinstruktur (hier gegenüber dem Virusstamm HRV 2).

### Influenza A Virus

Analog zu dem für die beiden Rhinoviren (HRV 2, HRV 14) beschriebenen Verfahren wird zunächst durch Zugabe einer Verdünnungsreihe wässriger Wirkstoff-Lösungen die Wirkstoffkonzentration ermittelt, bei der sich unter den gegebenen in vitro Testbedingungen kein Einfluss auf die verwendeten MDCK-Zellen ergibt. Die Wirkstoffkonzentration, bei der sich keine durch die Testsubstanzen bedingten, den Stoffwechsel der MDCK-Zellen reduzierenden Wirkungen ergeben, beträgt 0,005% (G/V). Die Testsubstanz wird in Verdünnungen (1:2, 1:4, 1:8, 1:16, 1:32) von der ermittelten, nicht zelltoxischen Substanzkonzentration von 0,005% (G/V) zu den infizierten Zellen zugegeben. Die antivirale Aktivität wird mit Hilfe des "high-throughput cytopathic effect inhibitory assay" (CPE/zpE) quantifiziert. Die Ergebnisse sind in Tabelle 4 zusammengestellt.

**Tabelle 4**

| Verdünnung (1/X) | ZpE-Hemmung [%] |
|---|---|
| 2 | 52,8 |
| 4 | 35,5 |
| 8 | 23,1 |
| 16 | 9,1 |

Die Ergebnisse zeigen beispielhaft für Oxymetazolin die antivirale Wirkung der α-Sympathomimetika gegenüber dem Virusstamm Influenza A (CPE Assay)

## Patentansprüche

1. Oxymetazolin und/oder Xylometazolin zur Verwendung zur antiviralen Prophylaxe und/oder antiviralen Behandlung viraler Erkrankungen ausgewählt aus der Gruppe bestehend aus Rhinitis acuta, Influenza, Parainfluenza, Otitis media und Sinusitis, wobei Oxymetazolin und/oder Xylometazolin antiviral wirksam sind.

2. Verbindungen zur Verwendung gemäß Anspruch 1, **dadurch gekennzeichnet, dass** das Arzneimittel zur topischen Anwendung bestimmt ist.

3. Verbindungen zur Verwendung gemäß Anspruch 2, **dadurch gekennzeichnet, dass** das Arzneimittel zur Anwendung auf Schleimhäute bestimmt ist.

4. Verbindungen zur Verwendung gemäß einem oder mehreren der Ansprüche 1 bis 3, **dadurch gekennzeichnet, dass** Oxymetazolin und/oder Xylometazolin in einem wässrigen Lösungsmittel enthalten ist/sind.

5. Verbindungen zur Verwendung nach Anspruch 4, **dadurch gekennzeichnet, dass** die wässrige Lösung mindestens einen Puffer, vorzugsweise Phosphat- oder Citratpuffer, enthält.

6. Verbindungen zur Verwendung nach Anspruch 4 und/oder 5, **dadurch gekennzeichnet, dass** die wässrige Lösung mindestens ein Konservierungsmittel, vorzugsweise Benzalkoniumchlorid, enthält.

## Claims

1. Oxymetazoline and/or xylometazoline for use for the antiviral prophylaxis and/or antiviral treatment of viral diseases selected from the group consisting of acute rhinitis, influenza, parainfluenza, otitis media and sinusitis, where oxymetazoline and/or xylometazoline have antiviral activity.

2. Compounds for use according to Claim 1, **characterised in that** the medicament is intended for topical use.

3. Compounds for use according to Claim 2, **characterised in that** the medicament is intended for use on mucous membranes.

4. Compounds for use according to one or more of Claims 1 to 3, **characterised in that** oxymetazoline and/or xylometazoline is (are) present in an aqueous solvent.

5. Compounds for use according to Claim 4, **characterised in that** the aqueous solution comprises at least one buffer, preferably phosphate or citrate buffer.

6. Compounds for use according to Claim 4 and/or 5, **characterised in that** the aqueous solution comprises at least one preservative, preferably benzalkonium chloride.

## Revendications

1. Oxymétazoline et/ou xylométazoline pour une utilisation dans la prophylaxie antivirale et/ou le traitement antiviral de maladies virales choisies dans le groupe constitué par la rhinite aiguë, la grippe, le parainfluenza, l'otite moyenne et la sinusite, où l'oxymétazoline et/ou la xylométazoline possèdent une activité antivirale.

2. Composés pour une utilisation selon la revendication 1, **caractérisés en ce que** le médicament est prévu pour une utilisation topique.

3. Composés pour une utilisation selon la revendication 2, **caractérisés en ce que** le médicament est prévu pour une utilisation sur les muqueuses.

4. Composés pour une utilisation selon l'une ou plusieurs parmi les revendications 1 à 3, **caractérisés en ce que** l'oxymétazoline et/ou la xylométazoline est ou sont présentes dans un solvant aqueux.

5. Composés pour une utilisation selon la revendication 4, **caractérisés en ce que** la solution aqueuse comprend au moins un tampon, préférablement un tampon phosphate ou citrate.

6. Composés pour une utilisation selon la revendication 4 et/ou 5, **caractérisés en ce que** la solution aqueuse comprend au moins un conservateur, préférablement le chlorure de benzalkonium.
